# EUROPÄISCHE PATENTSCHRIFT

(11) **EP 1 368 023 B1**
(45) Veröffentlichungstag und Bekanntmachung des Hinweises auf die Patenterteilung: **28.12.2005**
(21) Anmeldenummer: 02708327.8
(22) Anmeldetag: 18.02.2002
(51) Int. Cl.: A61K 31/31, A61P 13/10, A61K 9/70

(54) **VERWENDUNG VON BUPRENORPHIN ZUR THERAPIE DER HARNINKONTINENZ**
UTILIZATION OF BUPRENORPHINE IN URINARY INCONTINENCE THERAPY
UTILISATION DE BUPRENORPHINE POUR TRAITER L'INCONTINENCE D'URINE

(30) Priorität: 16.02.2001 DE 10107828; 18.09.2001 DE 20115429 U; 19.12.2001 DE 10162704
(43) Veröffentlichungstag der Anmeldung: 10.12.2003
(73) Patentinhaber: Grünenthal GmbH, 52078 Aachen (DE)
(72) Erfinder: BARTHOLOMÄUS, Johannes, 52080 Aachen (DE); CHRISTOPH, Thomas, 52080 Aachen (DE)
(86) Internationale Anmeldenummer: PCT/EP2002/001699
(87) Internationale Veröffentlichungsnummer: WO 2002/066031

(56) Entgegenhaltungen:
- EP-A- 0 819 438
- WO-A-00/23079
- WO-A-96/19975
- WO-A-96/27375
- US-A- 5 900 420
- MALINOVSKY J -M ET AL: "The urodynamic effects of intravenous opioids and ketoprofen in humans." ANESTHESIA & ANALGESIA, Bd. 87, Nr. 2, August 1998 (1998-08), Seiten 456-461, XP001064299 ISSN: 0003-2999 in der Anmeldung erwähnt
- MURRAY K: "ACUTE URINARY RETENTION ASSOCIATED WITH SUBLINGUAL BUPRENORPHINE" BRITISH MEDICAL JOURNAL, LONDON, GB, Bd. 286, Nr. 6367, 5. März 1983 (1983-03-05), Seiten 763-764, XP001064372 ISSN: 0267-0623
- LEANDER J D: "BUPRENORPHINE HAS POTENT KAPPA OPIOID RECEPTOR ANTAGONIST ACTIVITY" NEUROPHARMACOLOGY, PERGAMON PRESS, OXFORD, GB, Bd. 26, Nr. 9, 1987, Seiten 1445-1447, XP001064223 ISSN: 0028-3908

## Beschreibung

Die Erfindung betrifft die Verwendung von Buprenorphin zur Herstellung eines Arzneimittels zur Behandlung von vermehrtem Harndrang, erhöhter Miktionsfrequenz und/oder Harninkontinenz sowie entsprechende Arzneimittel und Verfahren zur Behandlung von vermehrtem Harndrang, erhöhter Miktionsfrequenz und/oder Harninkontinenz.

Harninkontinenz ist der unwillkürliche Harnabgang. Dieser tritt unkontrolliert auf, wenn der Druck innerhalb der Harnblase den Druck übersteigt, der zum Schließen des Harnleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z. B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Harnentleerung führt, der Sphinkter der Schließmuskelkomplex der Harnröhre. Es treten Mischformen dieser Inkontinenzarten sowie die sogenannte Überflußinkontinenz (z. B. bei benigner Prostatahyperplasie) oder Reflexinkontinenz (z. B. nach Rückenmarksschädigungen) auf. Näheres dazu findet sich bei Chutka, D. S. und Takahashi, P. Y., 1998, Drugs 560: 587-595.

Harndrang ist der auf Harnentleerung (Miktion) abzielende Zustand vermehrter Blasenmuskelspannung bei Annäherung an die Blasenkapazität (bzw. bei deren Überschreitung). Dabei umfaßt das Krankheitsbild Dranginkontinenz 1. vermehrten Harndrang 2. eine erhöhte Miktionsfrequenz und 3. die unwillkürliche Harninkontinenz als solche. Ursachen können u.a. Harnblasenentzündungen und neurogene Blasenstörungen sowie auch Blasentuberkulose sein. Es sind aber noch nicht alle Ursachen geklärt. Ein weiteres hier passendes Krankheitsbild ist die überaktive Blase (overactive bladder).

Vermehrter Harndrang, erhöhte Miktionsfrequenz wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Harndrang, erhöhte Miktionsfrequenz und insbesondere Harninkontinenz medikamentös mit Substanzen behandelt, die an den Reflexen des unteren Harntraktes beteiligt sind (Wein, A. J., 1998, Urology 51 (Suppl. 21): 43 - 47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Harnröhre oder des Blasenhalses erhöhen, zeigen Affinitäten zu α-Adrenorezeptoren wie Ephedrin, zu β-Adrenorezeptoren wie Clenbutarol oder sind Hormone wie Östradiol. Auch bestimmte Diarylmethylpiperazine und -piperidine sind für diese Indikation in der WO 93/15062 beschrieben. Ebenso wurde für Tramadol ein positiver Effekt auf die Blasenfunktion in einem Rattenmodell rhythmischer Blasenkontraktionen nachgewiesen (Nippon-Shinyaku, WO 98/46216).

Weiterhin gibt es Literatur, in der eine bekannte Nebenwirkung der Opiode, die Harnretention (Cousins und Mather, 1984, Anesthesiol. 61, 276-310), klinisch untersucht wird. Beispiele sind schwache Opioide wie Diphenoxylat (Fowler et al., 1987 J. Urol 138:735-738), starke Opioide wie Morphin (Malinovsky et al., 1998 a.a.O; Kontani und Kawabata, (1988); Jpn J Pharmacol. Sep;48(1):31) und Meperidin (Doyle and Briscoe, 1976 Br J Urol 48:329-335; Mohan et al., 1995, Int. J. Clin. Pharmacol. Therap. 33, 34-37) und sehr starke Opioide wie Fentanyl (Malinovsky et al., 1998 a.a.O; Drenger und Magora, 1989 Anesth Analg 69:348-353) oder auch gemischte Opioidagonisten / -antagonisten wie Pentazocin (Shimizu et al. (2000) Br. J. Pharmacol. 131 (3): 610 - 616; Mohan et al., 1995, Int. J. Clin. Pharmacol. Therap. 33, 34-37) und Nalbuphin (Malinovsky et al., 1998, a.a.O), Dextrorphan und Dextromethorphan (WO 9627375). Auch für Buprenorphin wurde ein verminderter Harndrang als unerwünschre Nebenwirkung festgestellt (Murray, K., British Medical Journal, London, GB, Bd. 286, Nr. 6367, 763-764). Diese Nebenwirkung wurde in einer Studie an Ratten dazu genutzt, die Affinität von Buprenorphin zum kappa-Opioid-Rezeptor näher zu charakterisieren (Leander, J. D., Neuropharmacology 1987, 26, 1445-1447). Allerdings erfolgten diese Untersuchungen, da es sich ja zumeist um humane Studien handelte, in analgetisch wirksamen Konzentrationen und in keinem dieser Fälle ist jemals ein positiver Effekt in der Behandlung von vermehrtem Harndrang bzw. Harninkontinenz berichtet worden. Vielmehr wird hier Harnretention festgestellt, was aber generell eine durchaus unerwünschte Wirkung ist und damit diese Verbindungen unattraktiv erscheinen läßt.

Bei den hier in Frage kommenden Indikationen ist weiter zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier - noch mehr als bei Analgetika - darauf zu achten, Nebenwirkungen zu vermeiden, will der Betroffene nicht ein Übel gegen das andere tauschen. Auch sind bei einer dauerhaften Harninkontinenzbehandlung auch analgetische Wirkungen weitgehend unerwünscht.

Aufgabe der vorliegenden Erfindung war es daher, Stoffe aufzufinden, die zur Behandlung von vermehrtem Harndrang, erhöhter Miktionsfrequenz bzw. Harninkontinenz hilfreich sind und bei den wirksamen Dosen bevorzugt gleichzeitig geringere Nebenwirkungen und/oder analgetische Wirkungen zeigen.

Überraschenderweise wurde nun gefunden, daß Buprenorphin bereits bei geringen Konzentrationen eine günstige Wirkung auf die Blasenfunktion insbesondere der Dranginkontinenz oder "overactive bladder", besitzt und gut zur Behandlung entsprechender Erkrankungsbilder geeignet ist.

Dementsprechend ist Erfindungsgegenstand die Verwendung von Buprenorphin auch in Form seiner Razemate, Enantiomere, Diastereomere, insbesondere in Form der Mischungen seiner Enantiomere oder Diastereomere oder in Form eines einzelnen Enantiomers oder Diastereomers; seiner Base und/oder Salze physiologisch verträglicher Säuren zur Herstellung eines Arzneimittels zur Behandlung von vermehrtem Harndrang, erhöhter Miktionsfrequenz und/oder Harninkontinenz, insbesondere der Dranginkontinenz oder "overactive bladder".

Überraschenderweise hatte sich herausgestellt, daß Buprenorphin in einem Modell, mit dem sich die beanspruchten Indikationen, insbesondere die Dranginkontinenz, abbilden lassen, hoch wirksam ist. Buprenorphine hebt im Modell die durch Oxy-Hämoglobin ausgelöste Detrusor-Überaktivität auf und beeinflußt die Blasenparameter entsprechend positiv. Gerade in einem Modell, das die Krankheitssymptome wie eben Dranginkontinenz, "overactive bladder" etc. deutlich zeigt, hat sich Buprenorphin damit bewährt.

Diese Wirkung ist auch insofern überraschend, da es auch zu Buprenorphin Untersuchungen in Bezug auf Harnretention und die Wirkung auf Blasen- und Urethra-Aktivität gibt (Murray K., 1983, Brit. Med. J. 286, 765-766; Drenger and Magora, 1989 Anesth Analg 69:348-353; Batra et al., 1996, Int. J. Clin. Pharmacol. Therap. 34, 309-311; Malinovsky et al., 1998 Anesth Analg 87:456-461), wobei aber die zum Teil etwas widersprüchlichen Ergebnisse gerade einem Einsatz von Buprenorphin in der Harninkontinenz widersprechen. So berichten Drenger wie auch Batra, daß eine epidurale Gabe von Buprenorphin im analgetisch wirksamen Bereich von 4 µg/kg (Batra) bzw. bei 2 µg/kg (Drenger [an Hunden]) keinen signifikanten Einfluß auf die Blasen- oder Urethrafunktion hat und empfehlen letztlich daher beide mangels Effekt gerade den Einsatz von Buprenorphin zur Schmerzbehandlung bei Patienten, bei denen Komplikationen im Harnbereich vermieden werden sollen. Umso erstaunlicher ist es, daß gerade Buprenorphin hier eine ausgesprochen günstige Wirkung in der Behandlung der Harninkontinenz zeigt.

Im anderen Extrem berichtet Malinovsky (Tabelle 4, S. 460), daß bei einer Gabe von 0,3 mg i.v. bei Patienten von etwa 70 kg Körpergewicht (4,3µg/kg) in 5 von 10 Fällen Harnretention auftritt und auch im von Murray vorgestellten Fall Harnretention nach sublingualer Einnahme von 400µg Buprenorphin (~ 5,7 µg/kg) eintritt. Abgesehen davon, daß Harnretention eine klar unerwünschte Wirkung ist, widersprechen sich hier die Daten und es decken bzw. überschneiden sich auch die berichteten Dosen, in denen sich im Ergebnis negative Wirkungen auf die Blase zeigen, mit solchen, in denen sich gar keine zeigen. Dabei ist es besonders irritierend, daß eine i.t. gegebene Menge von 4 µg/kg (Batra et al.) trotz des erheblich geringeren Verteilungsraumes und damit höherer Konzentration am Wirkort keinen Einfluß auf die Blasenfunktion hat, obwohl gerade IT-Gabe von Opioiden zu deutlicher Harninretention führen soll (Cousins und Mather 1984; Drenger und Magora 1989), während 4,3 µg/kg i.v. zu 50 % Harnretention führen sollen (Malinovsky et al.). Umso erstaunlicher war es, daß Buprenorphin trotz der von beiden Seiten negativen Literartur-Vorzeichen bei Harninkontinenz wirksam ist und insbesondere in einem Modell, das den Krankheitszustand abbildet.

Geeignete Salze im Sinne dieser Erfindung und in jeder der beanspruchten Verwendungen sind Salze des Buprenorphins mit anorganischen bzw. organischen Säuren und/oder einem Zuckeraustauschstoff wie Saccharin, Cyclamat oder Acesulfam. Besonders bevorzugt ist hier die freie Base, das Hydrochlorid, Stearat, Citrat oder Lactat, insbesondere die freie Base oder das Hydrochlorid.

Besonders bevorzugt ist es, wenn die Behandlung von vermehrtem Harndrang, erhöhter Miktionsfrequenz und/oder Harninkontinenz mit den unter erfindungsgemäßer Verwendung von Buprenorphin hergestellten Arzneimitteln mit einer Buprenorphin-Dosis unterhalb der Untergrenze der für die Schmerzbehandlung üblichen Dosis erfolgt.

Dabei ist es ganz besonders bevorzugt, wenn die Behandlung mit einer Buprenorphin-Menge < 300 µg oder < 4,3 µg/kg Körpergewicht, vorzugsweise zwischen 300 µg und 1 µg oder 4,3 µg/kg und 0,014 µg/kg, insbesondere zwischen 250 µg und 5 µg oder 3,6 µg/kg und 0,07 µg/kg, besonders bevorzugt zwischen 200 µg und 10 µg oder 2,8 µg/kg und 0,14 µg/kg erfolgt. Weiter gilt dabei bevorzugtermaßen, daß die angegebenen Buprenorphin-Mengen die maximalen bzw. minimalen Mengen einer Einzeldosis und/oder die pro Tag verabreichten maximalen oder minimalen Mengen sind.

Als in der Schmerztherapie üblich sind Dosierungen (i.m. oder i.v.) von 0,3 - 0,6 mg angegeben (Martindale (Ed. C. Parfitt), The complete drug reference, 32. Aufl., 1999, S. 22 f.). Entsprechend kann hier von ca. 300 µg als der Untergrenze der analgetischen Wirksamkeit ausgegangen werden. Die relative Dosierung in µ/kg wurde entsprechend auf einen Patienten mit einem durchschnittlichen Körpergewicht von 70 kg berechnet.

Angesichts der Daten beispielsweise von Batra et al. 1996, der mit einer Dosierung von 4µg/kg keine signifikanten Effekte an der Blase mit Buprenorphin im Menschen gesehen hatte, war es sehr überrraschend festzustellen, daß trotzdem bereits bei diesen und außerdem auch bei deutlich unter dieser unteren analgetischen Dosierung liegenden Buprenorphin-Mengen eine die Dranginkontinenz lindernde Wirkung des Buprenorphins im Tiermodell auftritt.

Es ist weiter besonders bevorzugt, wenn das unter erfindungsgemäßer Verwendung von Buprenorphin hergestellte Arzneimittel zur Behandlung von vermehrtem Harndrang, erhöhter Miktionsfrequenz und/oder Harninkontinenz eine verzögerte Freisetzung zeigt, vorzugsweise in Form einer Retard-Formulierung vorliegt.

Dies ist eine ganz besonders bevorzugte Ausführungsform der Erfindung, da die Behandlung der Harninkontinenz eine sehr langfristige Therapierung voraussetzt. Daher ist es sehr günstig, wenn das Arzneimittel eine verzögerte Freisetzung zeigt und der Wirkstoff entsprechend über einen längeren Zeitraum kontinuierlich abgegeben wird.

Dabei ist es zum einen eine bevorzugte Ausführungsform der Erfindung, wenn das Arzneimittel in der Form eines verzögert freisetzenden Partikels oder Implantats, insbesondere eines Kunststoff-Implantats oder -Partikels, vorliegt, wobei der Kunststoff vorzugsweise ausgewählt ist aus Polylactid, Polyglykolid oder einem Polylactid/Polyglykolid-Copolymer.

In dieser Ausführungsform wird das Buprenorphin vorzugsweise nichtkovalent an und in den Partikel oder das Implantat gebunden, der oder das nach Verabreichung meist unter Abbau der Trägermatrix des Partikels oder Implantats den Wirkstoff langsam, z.T. im Falle von Implantaten über Monate hinweg, und kontinuierlich in kleinen Mengen freisetzt. Gerade weil aber bei der Behandlung von Dranginkontinenz-Symptomen wie der Harninkontinenz mit Buprenorphin nur so überraschend niedrige Dosen notwendig sind und eine kontinuierlich langsame Freisetzung der Therapie nach vorliegenden Erkenntnissen entgegenkommt, hat sich herausgestellt, daß diese Form der Erfindung sehr günstig ist.

Eine ganz besonders bevorzugte Ausführungsform der Erfindung ist es zum anderen daher auch, wenn das hergestellte Arzneimittel ein transdermales therapeutisches System in Form eines Pflasters zur Verabreichung von Buprenorphin an die Haut ist. Auch oder gerade diese Form des unter erfindungsgemäßer Verwendung von Buprenorphin hergestellten Arzneimittels zur Behandlung von vermehrtem Harndrang, erhöhter Miktionsfrequenz und/oder Harninkontinenz zeigt nach den vorliegenden Erkenntnissen besonders günstige Eigenschaften in dieser Indikation bzw. diesen Indikationen. Günstigerweise setzen derartige Pflaster über einen Zeitraum von 3 oder auch 5 oder mehr Tagen kontinuierlich bestimmte leicht einstellbare Mengen an Buprenorphin frei, die auch sehr gering sein können (was überraschenderweise in dieser Indikation ausreicht), die dann über die Haut aufgenommen werden. Entsprechend geeignete Pflaster sind u.a. aus der EP 0 430 019 B1, der WO 98/36728 oder der WO96/19975 bekannt. Ein weiteres Pflaster zur transdermalen Anwendung von Buprenorphin ist in EP 819438 beschrieben. Wie in US 5900420 offenbart ist, kann Buprenorphin auch als transdermales Gel eingesetzt werden. Weiterhin sind auch orale Anwendungen von Buprenorphin beschrieben (WO 0023079).

Bevorzugt ist es, wenn das transdermale therapeutische System aus einer wirkstoffdurchlässigen Rückschicht, einer haftklebenden Reservoirschicht und einer wiederablösbaren Schutzschicht besteht. Dabei ist es weiter bevorzugt, wenn die Reservoirschicht 20-90 Gew.-% Polymermaterial, 0,1-30 Gew.-% Weichmacher, 0,1-20 Gew.-% Buprenorphin auch in Form seiner Razemate, Enantiomere, Diastereomere, insbesondere in Form der Mischungen seiner Enantiomere oder Diastereomere oder in Form eines einzelnen Enantiomers oder Diastereomers; seiner Base und/oder Salze physiologisch verträglicher Säuren, vorzugsweise in Form der Buprenorphinbase, und 0,1 -30 Gew.-% Lösungsmittel für Buprenorphin enthält, wobei das im System verbleibende Lösungsmittel für Buprenorphin in der Reservoirschicht vorzugsweise eine Verbindung mit mindestens einer sauren Gruppe ist.

Eine besonders bevorzugte Form des unter erfindungsgemäßer Verwendung von Buprenorphin hergestellten Arzneimittels zur Behandlung von vermehrtem Harndrang, erhöhter Miktionsfrequenz und/oder Harninkontinenz zeigt eine Freisetzungsrate des Buprenorphins zwischen 1 µg/h und 40 µg/h, vorzugsweise zwischen 2 µg/h und 35 µg/h, insbesondere zwischen 5 µg/h und 20 µg/h, bevorzugt zwischen 5 µg/h und 10 µg/h. Dies sind - wie sich herausgestellt hat - besonders günstige Freisetzungsraten für diese Indikationen.

Eine weitere bevorzugte Ausführungsform des unter erfindungsgemäßer Verwendung von Buprenorphin hergestellten Arzneimittels zur Behandlung von vermehrtem Harndrang, erhöhter Miktionsfrequenz und/oder Harninkontinenz in Form eines transdermalen therapeutischen Systems in Form eines Pflasters zur Verabreichung von Buprenorphin an die Haut ist es, wenn das transdermale therapeutische System eine Freisetzungsrate des Buprenorphins erster Ordnung über ein Dosierungsintervall von 72 h zeigt, so daß eine maximale Plasmakonzentration zwischen 20 pg/ml und 1052 pg/ml erreicht wird, und wenn bei der Behandlung das transdermale therapeutische System für ein weiteres Dosierungsinterval von mindestens 2 Tagen auf der Haut des Patienten verbleibt, während dessen das transdermale therapeutische System eine Freisetzungskinetik des Buprenorphins nullter Ordnung zeigt, so daß die Patienten während des zusätzlichen Dosierungsintervalls von mindestens 2 Tagen Analgesie erfahren.

Dabei ist es weiter bevorzugt, daß während des zusätzlichen Dosierungsintervalls von mindestens 2 Tagen eine relative durchschnittliche Freisetzungsrate von zwischen 0,3 µg/h und 21 µg/h, vorzugsweise zwischen 0,3 µg/h und 9 µg/h oder zwischen 13 µg/h und 21 µg/h, insbesondere zwischen 0,3 µg/h und 0,6 µg/h, zwischen 0,7 µg/h und 1 µg/h, zwischen 2 µg/h und 4 µg/h, zwischen 4 µg/h und 7 µg/h oder zwischen 5 µg/h und 9 µg/h aufrechterhalten wird.

Ebenfalls bevorzugt ist diese Form dann, wenn das entsprechende transdermales therapeutische System eine Freisetzungsrate des Buprenorphins erster Ordnung über ein Dosierungsintervall von 72 h zeigt, so daß ca. 72 h nach Anwendung dieses transdermalen therapeutischen Systems eine durchschnittliche Plasmakonzentration zwischen 20 pg/ml und 1052 pg/ml, vorzugsweise zwischen 85 pg/ml und 263 pg/ml, insbesondere zwischen 20 pg/ml und 66 pg/ml, zwischen 42 pg/ml und 132 pg/ml, zwischen 169 pg/ml und 526 pg/ml, zwischen 254 pg/ml und 789 pg/ml oder zwischen 339 pg/ml und 1052 pg/ml erreicht wird.

Günstig sind auch entsprechende Transdermale therapeutische Syteme, bei denen ca. 72 h nach Anwendung dieses transdermalen -therapeutischen Systems eine durchschnittliche Plasmakonzentration zwischen 20 pg/ml und 1052 pg/ml und während des zusätzlichen Dosierungsintervalls von mindestens 2 Tagen eine relative durchschnittliche Freisetzungsrate von zwischen 0,3 µg/h und 9 µg/h vorliegt oder eine durchschnittliche Plasmakonzentration zwischen 85 pg/ml und 263 pg/ml und eine relative durchschnittliche Freisetzungsrate von zwischen 13 µg/h und 21 µg/h oder eine durchschnittliche Plasmakonzentration zwischen 20 pg/ml und 66 pg/ml und eine relative durchschnittliche Freisetzungsrate von zwischen 0,3 µg/h und 0,6 µg/h oder eine durchschnittliche Plasmakonzentration zwischen 42 pg/ml und 132 pg/ml und eine relative durchschnittliche Freisetzungsrate von zwischen 0,7 µg/h und 1 µg/h oder eine durchschnittliche Plasmakonzentration zwischen 169 pg/ml und 526 pg/ml und eine relative durchschnittliche Freisetzungsrate von zwischen 2 µg/h und 4 µg/h oder eine durchschnittliche Plasmakonzentration zwischen 254 pg/ml und 789 pg/ml und eine relative durchschnittliche Freisetzungsrate von zwischen 4 µg/h und 7 µg/h oder eine durchschnittliche Plasmakonzentration zwischen 339 pg/ml und 1052 pg/ml und eine relative durchschnittliche Freisetzungsrate von zwischen 5 µg/h und 9 µg/h.

Weiter bevorzugt ist diese Ausführungsform dann, wenn das transdermale therapeutische System mindestens 5 Tage auf der Haut des Patienten verbleibt.

Auch wenn Buprenorphin in der erfindungsgemässen Verwendung lediglich geringe bis gar keine Nebenwirkungen zeigt, kann es beispielsweise zur Vermeidung von bestimmten Formen der Abhängigkeit auch von Vorteil sein, neben diesen Verbindungen auch Morphinantagonisten, insbesondere Naloxon, Naltrexon und/oder Levallorphan, zu verwenden.

Die Erfindung umfaßt ebenfalls Arzneimittel zur Behandlung von vermehrtem Harndrang, erhöhter Miktionsfrequenz und/oder Harninkontinenz, die als Wirkstoff wenigstens Buprenorphin auch in Form seiner Razemate, Enantiomere, Diastereomere, insbesondere in Form der Mischungen seiner Enantiomere oder Diastereomere oder in Form eines einzelnen Enantiomers oder Diastereomers; seiner Basen und/oder Salze physiologisch verträglicher Säuren sowie gegebenenfalls Zusatz- und/oder Hilfsstoffe enthalten.

Generell können diese erfindungsgemäßen Arzneimittel wie auch die oben beschriebenen unter erfindungsgemäßer Verwendung von Buprenorphin hergestellten Arzneimittel zur Behandlung von vermehrtem Harndrang, erhöhter Miktionsfrequenz und/oder Harninkontinenz zum einen Zusatz- und/oder Hilfsstoffe enthalten, zum anderen in den verschiedensten bekannten Arzneimittelformen vorliegen.

Geeignete Zusatz- und/oder Hilfsstoffe im Sinne dieser Erfindung sind alle dem Fachmann aus dem Stand der Technik bekannten Stoffe zur Erreichung galenischer Formulierungen. Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum.

Aus bestimmten Zubereitungsformen kann Buprenorphin verzögert freigesetzt werden. Beispiele sind retardierende Tablettenformen aber insbesondere auch die Anwendung von Buprenorphin in einem Depot in gelöster Form, einer Trägerfolie oder einem Pflaster, gegebenenfalls unter Zusatz von die Hautpenetration fördernden Mitteln als geeignete Beispiele für geeignete perkutane Applikationsformen wie auch verzögert freisetzende Partikel oder Implantate.

Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden.

Eine besonders bevorzugte Form des erfindungsgemäßen Arzneimittels liegt vor, wenn das Arzneimittel eine verzögerte Freisetzung zeigt, vorzugsweise in Form einer Retard-Formulierung vorliegt, insbesondere
in der Form eines verzögert freisetzenden Partikels oder Implantats, vorzugsweise Kunststoff-Implantats oder -Partikels, vorliegt, wobei der Kunststoff vorzugsweise ausgewählt ist aus Polylactid bzw. einem Polylactid/Polyglykolid-Copolymer
oder
ein transdermales therapeutisches System in Form eines Pflasters zur Verabreichung von Buprenorphin an die Haut ist.

Im übrigen gelten für das erfindungsgemäße Arzneimittel die gleichen bevorzugten Ausführungsformen wie sie oben bereits für unter erfindungsgemäßer Verwendung von Buprenorphin hergestellte Arzneimittel zur Behandlung von vermehrtem Harndrang, erhöhter Miktionsfrequenz und/oder Harninkontinenz beschrieben wurden.

Die Herstellung der erfindungsgemäßen Arzneimittel und pharmazeutischen Zusammensetzungen kann mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Aber auch andere Herstellungsarten insbesondere für moderne Arzneimittelformen sind denkbar und bekannt.

Weiter betrifft die Erfindung auch ein Verfahren zur Behandlung von vermehrtem Harndrang, erhöhter Miktionsfrequenz bzw. Harninkontinenz, bei dem Buprenorphin in Form seiner Razemate, Enantiomere, Diastereomere, insbesondere in Form der Mischungen seiner Enantiomere oder Diastereomere oder in Form eines einzelnen Enantiomers oder Diastereomers; seiner Basen und/oder Salze physiologisch verträglicher Säuren, verwendet wird.

Die folgenden Beispiele sollen die Erfindung erläutern.

### Beispiele

### Beispiel 1: Testsystem Cystometrie an der wachen naiven Ratte

Es wurden cystometrische Untersuchungen an naiven, weiblichen Sprague-Dawley-Ratten nach der Methode von Ishizuka et al. ((1997), Naunyn-Schmiedeberg's Arch. Pharmacol. 355: 787 - 793) durchgeführt Drei Tage nach Implantation von Blasen- und venösen Kathetern wurden die Tiere im wachen Zustand, frei beweglich untersucht. Der Blasenkatheter wurde an einem Druckaufnehmer und eine Injektionspumpe angeschlossen. Die Tiere wurden in Stoffwechselkäfige gesetzt, die die Messung des Harnvolumens ermöglichten. Physiologische Kochsalzlösung wurde in die entleerte Blase infundiert (10 ml/Std.) und Blasendruck und Miktionsvolumen kontinuierlich aufgezeichnet Nach einer Stabilisierungsphase wurde eine 20minütige Phase aufgezeichnet, die durch normale, reproduzierbare Miktionszyklen gekennzeichnet war. Es wurden unter anderem die folgenden Parameter bestimmt:
■ Schwellendruck (threshold pressure TP, Blasendruck unmittelbar vor Miktion),
■ Blasenkapazität (bladder capacity BC, Restvolumen nach vorhergehender Miktion plus Volumen der infudierten Lösung während der Füllungsphase),
■ Interkontraktionsintervall (inter-contraction interval (ICI), das Zeitintervall zwischen den Miktionen).

Eine Erhöhung des Schwellendrucks (TP) zeigt eine wichtige therapeutische Wirkung bei einer der erfindungsgemässen Indikationen an. Auch das Interkontraktionsintervall (ICI) ist ein wichtiger Parameter zur Messung der physiologischen Wirksamkeit eines Stoffes in der Behandlung der Harninkontinenz, ebenso wie die Blasenkapazität (BC). Dabei ist es für eine Wirksamkeit aufgrund der sehr heterogenen Ursachen für die Symptomatik dieser Erkrankungsbilder nicht nötig, alle drei Parameter positiv zu beeinflussen. Es genügt daher völlig, wenn nur in einem dieser Parameter eine posive Wirkung festzustellen ist, um in der Harninkontinenz, erhöhter Miktionsfrequenz oder vermehrtem Harndrang einsetzbar zu sein.

Nach der Aufzeichnung von drei reproduzierbaren Miktionszyklen als Vorwert wurden 10 µg/kg Buprenorphin im Vehikel = 0,9 % NaCl i.v. appliziert und die Wirkung auf die cystometrischen Parameter 90 bis 120 Minuten aufgezeichnet. Im Wirkmaximum wurde der Mittelwert von 3 Miktionszyklen bestimmt und als prozentuale Veränderung gegenüber dem Vorwert dargestellt (Tabelle 1).

Die eingesetzte Konzentration entspricht dem ED₅₀ in einem bekannten Analgesiemodell für Ratten, dem Tail Flick.

**Tabelle 1:**

| **Buprenorphin** | **TP** threshold pressure | **BC** bladder capacity | **ICI** inter-contraction interval |
|---|---|---|---|
| **0,01 mg/kg iv** | +69,9% | +3,6% | +10,9% |
| (n=6) | ** | | |
| Beeinflussung der cystometrischen Parameter durch Buprenorphin (Veränderung zum Vorwert [%]); n entspricht der Anzahl der im Versuch eingesetzten Tiere. Signifikanz (Student T-Test): * p < 0.05; ** p < 0.01 *** p < 0.001. | | | |

Buprenorphin zeigt gerade beim TP eine positive Wirkung auf die Blasenregulation und ist damit prinzipiell geeignet zur Behandlung der Harninkontinenz. Allerdings war die eingesetzte Konzentration, die analgetisch wirksam ist, offenbar zu hoch, da bei 2 der 6 Tiere bereits Tropf-Inkontinenz auftrat. Bei zwei niedrigeren Konzentrationen, 0,001 mg/kg i.v. und 0,005 mg/kg i.v. trat bei n=6 eine Steigerung des TP von + 27,6 % bzw. + 37,5% auf.

### Beispiel 2: Testsystem Cystometrie an der wachen geschädigten Ratte

Dieses Modell simuliert die Dranginkontinenz im Tiermodell; das eingesetzte Oxyhemoglobin (OxyHb) induziert eine Blasenüberaktivität.

Es wurden cystometrische Untersuchungen an naiven, weiblichen Sprague-Dawley-Ratten nach der Methode von Pandita et al. (J. Urol. 2000, 164:545-550) durchgeführt. Drei Tage nach Implantation von Blasen- und venösen Kathetern wurden die Tiere im wachen Zustand, frei beweglich untersucht. Der Blasenkatheter wurde an einem Druckaufnehmer und eine Injektionspumpe angeschlossen. Die Tiere wurden in Stoffwechselkäfige gesetzt, die die Messung des Harnvolumens ermöglichten. Physiologische Kochsalzlösung wurde in die entleerte Blase infundiert (10 ml/Std.) und Blasendruck und Miktionsvolumen kontinuierlich aufgezeichnet. Nach einer Stabilisierungsphase wurde eine 20minütige Phase aufgezeichnet, die durch normale, reproduzierbare Miktionszyklen gekennzeichnet war. Es wurden unter anderem die folgenden Parameter bestimmt:
■ Schwellendruck (threshold pressure TP, Blasendruck unmittelbar vor Miktion),
■ Blasenkapazität (bladder capacity BC, Restvolumen nach vorhergehender Miktion plus Volumen der infudierten Lösung während der Füllungsphase),
■ Interkontraktionsintervall (inter-contraction interval (ICI), das Zeitintervall zwischen den Miktionen).
■ Miktionsdruck (micturition pressure MP, maximaler Blasendruck während einer Miktion).

Eine Erhöhung des Schwellendrucks (TP) zeigt eine wichtige therapeutische Wirkung bei einer der erfindungsgemässen Indikationen an. Auch das Interkontraktionsintervall (ICI) ist ein wichtiger Parameter zur Messung der physiologischen Wirksamkeit eines Stoffes in der Behandlung der Harninkontinenz, ebenso wie die Blasenkapazität (BC). Dabei ist es für eine Wirksamkeit aufgrund der sehr heterogenen Ursachen für die Symptomatik dieser Erkrankungsbilder nicht nötig, alle Parameter positiv zu beeinflussen. Es genügt daher völlig, wenn nur in einem dieser Parameter eine positive Wirkung festzustellen ist, um in der Harninkontinenz, erhöhter Miktionsfrequenz oder vermehrtem Harndrang einsetzbar zu sein.

Nach der Aufzeichnung von drei reproduzierbaren Miktionszyklen als Vorwert wurden 2.5x10⁻⁴M Oxyhämoglobin im Vehikel = 0,9% NaCl in die Blase infundiert. Die Wirkung auf die cystometrischen Parameter wurden etwa 20 Minuten aufgezeichnet. Im Wirkmaximum wurde der Mittelwert von 3 Miktionszyklen bestimmt und als prozentuale Veränderung gegenüber dem Vorwert dargestellt (Tabelle 2). Die Behandlung mit Oxyhämoglobin induziert eine charakteristische Veränderung der cystometrischen Parameter mit einer Erhöhung des Miktionsdrucks, einer Erniedrigung der Blasenkapazität und einer Verringerung des Interkontraktionsintervals. Diese Veränderungen bilden die Veränderungen ab, die bei Patienten mit Dranginkontinenz gefunden werden.

Die Applikation von 5 µg/kg Buprenorphin im Vehikel = 0,9 % NaCl i.v. vor der Applikation von Oxyhämoglobin ist in der Lage die Veränderungen, die durch Oxyhämoglobin induziert werden, zu unterdrücken und darüber hinaus noch einen Anstieg des Schwellendrucks zu induzieren (Tabelle 2).

**Tabelle 2:**

| | **MP** Micturition pressure [cm H₂O] | **TP** threshold pressure [cm H₂O] | **BC** bladder capacity [ml] | **ICI** intercontraction interval [min] |
|---|---|---|---|---|
| **OxyHb** | | | | |
| **2,5×10**^{**-4**}**M iv** | v: 59 ± 8 | v: 8,72 ± 1,31 | v: 0.92 ± 0,10 | v: 4.96 ± 0,33 |
| | h: 97 ± 5 | h: 9,84 ± 1,56 | h: 0,65 ± 0,06 | h: 3,33 ± 0,18 |
| (n=5) | Dif.:+64,4% | Diff.: +12,8% | Diff.: -29,3% | Diff.: -32,9% |
| | ** | | ** | ** |

| **OxyHb + Buprenorphin** | | | | |
|---|---|---|---|---|
| **OxyHb:** | v: 54 ± 9 | v: 9,07 ± 1,29 | v: 1, 19 ± 0,12 | v: 6.72 ± 0,73 |
| **2,5×10**^{**-4**}**M** | h: 37 ± 8 | h: 14,28± 2,53 | h: 1,17 ± 0,13 | h: 6,70 ± 0,88 |
| **Buprenorphin:** | Diff.: -31,5% | Diff.: +57,4 % | Diff.: -1,7 % | Diff.: -0,3 % |
| **0,005 mg/kg iv** | * | * | | |
| (n=6) | | | | |
| Beeinflussung der cystometrischen Parameter durch Oxyhämoglobin (OxyHb) mit und ohne vorherige Gabe von Buprenorphin. Angegeben sind Durchschnittswerte mit Standardabweichungen vor (v) und nach (h) Anwendung der Substanzen sowie die Veränderung (Diff.) im Vergleich zum Vorwert [%]; n entspricht der Anzahl der im Versuch eingesetzten Tiere. Signifikanz (Student T-Test):* p < 0.05; ** p < 0.01; *** p < 0.001. | | | | |

Es ist zu erkennen, daß OxyHb die Blasenparameter deutlich im Sinne einer Dranginkontinenz negativ beeinflußt. Diese negative Beeinflußung wird durch Buprenorphin aufgehoben und sogar verbessert. So sinkt der Miktionsdruck im Vergleich zu der durch OxyHb ausgelösten Dranginkontinenz und auch im Vergleich zur unbehandelten Kontrolle signifikant. Weiter normalisiert Buprenorphin in diesem Dranginkontinenzmodell das Interkontraktionsintervall und die Blasenkapazität vollkommen und bewirkt weiter eine signifikante und deutliche Erhöhung des Schwellendrucks.

Damit ist der Beweis angetreten, daß Buprenorphin, insbesondere im Bereich der Dranginkontinenz, für die das OxyHb-Modell als Standardmodell steht, eine hervorragende Wirkung zeigt und zwar auch bei Schädigung, also im Krankheitsfall.

### Beispiel 3: Transdermale Formulierung:

Es wird ein transdermales Apllikationsystem gemäß Beispiel 1 der WO 98/36728 formuliert.

1,139 g einer Polyacrylat-Lösung von 47,83 Gew.-% mit selbstvernetzenden Acrylat-Copolymeren enthaltend 2-Ethylhexylacrylat, Vinyl-Acetat, Acrylsäure (Lösungsmittel: Ethylacetat : Heptan : Isopropanol : Toluol : Acetylacetonat im Verhältnis 37:26:26:4:1), 100 g Laevulininsäure, 150 g Oleyloleat, 100 g Polyvinylpyrrolidon, 150 g Ethanol, 200 g Ethylacetat und 100 g Buprenorphinbase wurden homogenisiert. Die Mischung wurde für ca. 2 Std. gerührt und dann visuell untersucht um festzustellen, ob alle festen Substanzen aufgelöst wurden. Man muß die Verdampfungsverluste durch erneutes Wiegen kontrollieren und falls nötig das Lösungsmittel mit Hilfe von Ethylacetat ausgleichen. Danach wird die Mischung auf eine 420 mm breite transparente Polyester-Folie gebracht, so daß das Oberflächengewicht der getrockneten Schicht 80g/m² beträgt. Die Polyesterfolie dient als Schutzschicht und kann durch die Behandlung mit Silicon wieder aufgelöst werden. Das Lösungsmittel wird durch erhitzte Luft, die über eine feuchte Strecke geführt wird, entfernt. Durch diese Wärmebehandlung verdampfen nicht nur die Lösungsmittel sondern es schmilzt auch die Laevulininsäure. Danach wird der Siegelfilm mit einer Polyesterfolie von 15 µ Dicke abgedeckt. Eine Fläche von 16 cm² wird mit Hilfe eines geeigneten Schneidwerkzeuges ausgeschnitten und die Ränder, die zwischen den einzelnen Gegenständen stehengeblieben sind, werden entfernt.

Um die nominelle Freisetzungsgeschwindigkeit von ca. 25 µg/h zu erreichen, ist die Gesamtmenge an Buprenorphin im Transdermal-Pflaster ca. 10 mg, die aktive Oberfläche sind ca. 12,5 cm² und die Größe des Pflasters beispielsweise ca. 30,6 cm².

## Patentansprüche

1. Verwendung von Buprenorphin auch in Form seiner Razemate, Enantiomere, Diastereomere, insbesondere in Form der Mischungen seiner Enantiomere oder Diastereomere oder in Form eines einzelnen Enantiomers oder Diastereomers; seiner Base und/oder Salze physiologisch verträglicher Säuren zur Herstellung eines Arzneimittels zur Behandlung von vermehrtem Harndrang, erhöhter Miktionsfrequenz und/oder Harninkontinenz, insbesondere der Dranginkontinenz oder "overactive bladder".

2. Verwendung gemäß Anspruch 1, **dadurch gekennzeichnet, daß** die Behandlung mit einer Buprenorphin-Dosis unterhalb der Untergrenze der für die Schmerzbehandlung üblichen Dosis erfolgt.

3. Verwendung gemäß Anspruch 1, **dadurch gekennzeichnet, daß** die Behandlung mit einer Buprenorphin-Menge < 300 µg oder < 4,3 µg/kg Körpergewicht, vorzugsweise zwischen 300 µg und 1 µg oder 4,3 µg/kg und 0,014 µg/kg, insbesondere zwischen 250 µg und 5 µg oder 3,6 µg/kg und 0,07 µg/kg, besonders bevorzugt zwischen 200 µg und 10 µg oder 2,8 µg/kg und 0,14 µg/kg erfolgt.

4. Verwendung gemäß Anspruch 3, **dadurch gekennzeichnet, daß** die Buprenorphin-Menge die maximale bzw. minimale Menge einer Einzeldosis und/oder die pro Tag verabreichte maximale oder minimale Menge ist.

5. Verwendung gemäß einem der Ansprüche 1 bis 4, **dadurch gekennzeichnet, daß** das Arzneimittel eine verzögerte Freisetzung zeigt, vorzugsweise in Form einer Retard-Formulierung vorliegt.

6. Verwendung gemäß Anspruch 5, **dadurch gekennzeichnet, daß** das Arzneimittel in der Form eines verzögert freisetzenden Partikels oder Implantats, insbesondere Kunststoff-Implantats oder - Partikels, vorliegt, wobei der Kunststoff vorzugsweise ausgewählt ist aus Polylactid, Polyglykolid oder einem Polylactid/Polyglykolid-Copolymer.

7. Verwendung gemäß Anspruch 5, **dadurch gekennzeichnet, daß** das hergestellte Arzneimittel ein transdermales therapeutisches System in Form eines Pflasters zur Verabreichung von Buprenorphin an die Haut ist.

8. Verwendung gemäß Anspruch 7, **dadurch gekennzeichnet, daß** das transdermale therapeutische System aus einer wirkstoffdurchlässigen Rückschicht, einer haftklebenden Reservoirschicht und einer wiederablösbaren Schutzschicht besteht.

9. Verwendung gemäß Anspruch 8, **dadurch gekennzeichnet, daß** die Reservoirschicht 20-90 Gew.-% Polymermaterial, 0,1-30 Gew.-% Weichmacher, 0,1-20 Gew.-% Buprenorphin auch in Form seiner Razemate, Enantiomere, Diastereomere, insbesondere in Form der Mischungen seiner Enantiomere oder Diastereomere oder in Form eines einzelnen Enantiomers oder Diastereomers; seiner Base und/oder Salze physiologisch verträglicher Säuren, vorzugsweise in Form der Buprenorphinbase, und 0,1 -30 Gew.-% Lösungsmittel für Buprenorphin enthält, wobei das im System verbleibende Lösungsmittel für Buprenorphin in der Reservoirschicht vorzugsweise eine Verbindung mit mindestens einer sauren Gruppe ist.

10. Verwendung gemäß einem der Ansprüche 1 bis 9, **dadurch gekennzeichnet, daß** das Arzneimittel eine Freisetzungsrate des Buprenorphins zwischen 1 µg/h und 40 µg/h, vorzugsweise zwischen 2 µg/h und 35 µg/h, insbesondere zwischen 5 µg/h und 20 µg/h, bevorzugt zwischen 5 µg/h und 10 µg/h zeigt.

11. Verwendung gemäß einem der Ansprüche 7 bis 9, **dadurch gekennzeichnet, daß** das transdermale therapeutische System eine Freisetzungsrate des Buprenorphins erster Ordnung über ein Dosierungsintervall von 72 h zeigt, so daß eine maximale Plasmakonzentration zwischen 20 pg/ml und 1052 pg/ml erreicht wird, und daß bei der Behandlung das transdermale therapeutische System für ein weiteres Dosierungsinterval von mindestens 2 Tagen auf der Haut des Patienten verbleibt, während dessen das transdermale therapeutische System eine Freisetzungskinetik des Buprenorphins nullter Ordnung zeigt, so daß die Patienten während des zusätzlichen Dosierungsintervalls von mindestens 2 Tagen Analgesie erfahren.

12. Verwendung gemäß Anspruch 11, **dadurch gekennzeichnet, daß** während des zusätzlichen Dosierungsintervalls von mindestens 2 Tagen eine relative durchschnittliche Freisetzungsrate von zwischen 0,3 µg/h und 21 µg/h, vorzugsweise zwischen 0,3 µg/h und 9 µg/h oder zwischen 13 µg/h und 21 µg/h, insbesondere zwischen 0,3 µg/h und 0,6 µg/h, zwischen 0,7 µg/h und 1 µg/h, zwischen 2 µg/h und 4 µg/h, zwischen 4 µg/h und 7 µg/h oder zwischen 5 µg/h und 9 µg/h aufrechterhalten wird.

13. Verwendung gemäß einem der Ansprüche 11 oder 12, **dadurch gekennzeichnet, daß** das transdermales therapeutische System eine Freisetzungsrate des Buprenorphins erster Ordnung über ein Dosierungsintervall von 72 h zeigt, so daß ca. 72 h nach Anwendung dieses transdermalen therapeutischen Systems eine durchschnittliche Plasmakonzentration zwischen 20 pg/ml und 1052 pg/ml, vorzugsweise zwischen 85 pg/ml und 263 pg/ml, insbesondere zwischen 20 pg/ml und 66 pg/ml, zwischen 42 pg/ml und 132 pg/ml, zwischen 169 pg/ml und 526 pg/ml, zwischen 254 pg/ml und 789 pg/ml oder zwischen 339 pg/ml und 1052 pg/ml erreicht wird.

14. Verwendung gemäß einem der Ansprüche 11 bis 13, **dadurch gekennzeichnet, daß** das transdermale therapeutische System mindestens 5 Tagen auf der Haut des Patienten gelassen wird.

15. Verwendung gemäß einem der Ansprüche 1 bis 14, **dadurch gekennzeichnet, daß** das Arzneimittel neben Buprenorphin einen Morphinantagonisten, insbesondere Naloxon, Naltrexon und/oder Levallorphan, enthält.

16. Verwendung gemäß einem der Ansprüche 1 bis 15, **dadurch gekennzeichnet, daß** das Arzneimittel Buprenorphin in Form der freien Base, des Hydrochlorids, des Stearats, des Citrats oder Lactats, insbesondere der freien Base oder des Hydrochlorids, enthält.

## Claims

1. Use of buprenorphine, also in the form of its racemates, enantiomers and diastereomers, in particular in the form of mixtures of its enantiomers or diastereomers or in the form of an individual enantiomer or diastereomer; its base and/or salts of physiologically acceptable acids, for the preparation of a medicament for treatment of an increased urge to urinate, an increased frequency of micturition and/or urinary incontinence, in particular urgency incontinence or "overactive bladder".

2. Use according to claim 1, **characterized in that** the treatment is carried out with a buprenorphine dose below the lower limit of the conventional dose for pain treatment.

3. Use according to claim 1, **characterized in that** the treatment is carried out with an amount of buprenorphine of < 300 *µ*g or < 4.3 *µ*g/kg of body weight, preferably between 300 *µ*g and 1 *µ*g or 4.3 *µ*g/kg and 0.014 *µ*g/kg, in particular between 250 *µ*g and 5 *µ*g or 3.6 *µ*g/kg and 0.07 *µ*g/kg, particularly preferably between 200 *µ*g and 10 *µ*g or 2.8 *µ*g/kg and 0.14 *µ*g/kg.

4. Use according to claim 3, **characterized in that** the amount of buprenorphine is the maximum or minimum amount of an individual dose and/or the maximum or minimum amount administered per day.

5. Use according to one of claims 1 to 4, **characterized in that** the medicament shows a delayed release, and is preferably present in the form of a sustained release formulation.

6. Use according to claim 5, **characterized in that** the medicament is in the form of a delayed-release particle or implant, in particular an implant or particle of synthetic material, the synthetic material preferably being chosen from polylactide, polyglycollide or a polylactide/polyglycollide copolymer.

7. Use according to claim 5, **characterized in that** the medicament prepared is a transdermal therapeutic system in the form of a patch for administration of buprenorphine to the skin.

8. Use according to claim 7, **characterized in that** the transdermal therapeutic system comprises a backing layer which is permeable to active compounds, an adhesive reservoir layer and a re-detachable protective layer.

9. Use according to claim 8, **characterized in that** the reservoir layer contains 20-90 wt.% of polymer material, 0.1-30 wt.% of plasticizer, 0.1-20 wt.% of buprenorphine, also in the form of its racemates, enantiomers or diastereomers, in particular in the form of mixtures of its enantiomers or diastereomers or in the form of an individual enantiomer or diastereomer; its base and/or salts of physiologically acceptable acids, preferably in the form of buprenorphine base, and 0.1-30 wt.% of a solvent for buprenorphine, the solvent for buprenorphine in the reservoir layer which remains in the system preferably being a compound with at least one acid group.

10. Use according to one of claims 1 to 9, **characterized in that** the medicament shows a release rate of the buprenorphine of between 1 µg/h and 40 µg/h, preferably between 2 µg/h and 35 µg/h, in particular between 5 µg/h and 20 µg/h, preferably between 5 µg/h and 10 µg/h.

11. Use according to one of claims 7 to 9, **characterized in that** the transdermal therapeutic system has a release rate of the buprenorphine of the first order over a dosage interval of 72 h, so that a maximum plasma concentration of between 20 pg/ml and 1,052 pg/ml is achieved, and **in that**, during the treatment, the transdermal therapeutic system remains on the skin of the patient for a further dosage interval of at least 2 days, during which the transdermal therapeutic system shows release kinetics of the buprenorphine of zero order, so that the patients experience analgesia during the additional dosage interval of at least two days.

12. Use according to claim 11, **characterized in that** during the additional dosage interval of at least 2 days, a relative average release rate of between 0.3 *µ*g/h and 21 *µ*g/h, preferably between 0.3 *µ*g/h and 9 *µ*g/h or between 13 *µ*g/h and 21 *µ*g/h, in particular between 0.3 *µ*g/h and 0.6 *µ*g/h, between 0.7 *µ*g/h and 1 *µ*g/h, between 2 *µ*g/h and 4 *µ*g/h, between 4 *µ*g/h and 7 *µ*g/h or between 5 *µ*g/h and 9 *µ*g/h is maintained.

13. Use according to one of claims 11 or 12, **characterized in that** the transdermal therapeutic system has a release rate of the buprenorphine of the first order over a dosage interval of 72 h, so that approx. 72 h after use of this transdermal therapeutic system an average plasma concentration of between 20 pg/ml and 1,052 pg/ml, preferably between 85 pg/ml and 263 pg/ml, in particular between 20 pg/ml and 66 pg/ml, between 42 pg/ml and 132 pg/ml, between 169 pg/ml and 526 pg/ml, between 254 pg/ml and 789 pg/ml or between 339 pg/ml and 1,052 pg/ml is achieved.

14. Use according to one of claims 11 to 13, **characterized in that** the transdermal therapeutic system is left on the skin of the patient for at least 5 days.

15. Use according to one of claims 1 to 14, **characterized in that** the medicament comprises a morphine antagonist, in particular naloxone, naltrexone and/or levallorphan, in addition to buprenorphine.

16. Use according to one of claims 1 to 15, **characterized in that** the medicament comprises buprenorphine in the form of the free base, the hydrochloride, the stearate, the citrate or lactate, in particular the free base or the hydrochloride.

## Revendications

1. Utilisation de buprénorphine, également sous forme de ses racémates, de ses énantiomères, ses diastéréoisomères, en particulier sous forme des mélanges de ses énantiomères ou diastéréoisomères ou sous forme d'un énantiomère ou d'un diastéréoisomère individuel ; sous forme de sa base et/ou sous forme de sels d'acides acceptables du point de vue physiologique, pour la production d'un médicament destiné au traitement de la répétition des besoins impérieux d'uriner, de l'élévation de la fréquence de la miction et/ou de l'incontinence d'urine, en particulier de l'incontinence par besoin impérieux ou "overactive bladder".

2. Utilisation suivant la revendication 1, **caractérisée en ce que** le traitement est effectué avec une dose de buprénorphine en deçà de la limite inférieure de la dose habituelle utilisée pour le traitement de la douleur.

3. Utilisation suivant la revendication 1, **caractérisée en ce que** le traitement est effectué avec une quantité de buprénorphine inférieure à 300 µg ou inférieure à 4,3 µg/kg de poids corporel, avantageusement entre 300 µg et 1 µg ou entre 4,3 µg/kg et 0,014 µg/kg, en particulier entre 250 µg et 5 µg ou entre 3, 6 µg/kg et 0, 07 µg/kg, notamment entre 200 µg et 10 µg ou entre 2,8 µg/kg et 0,14 µg/kg.

4. Utilisation suivant la revendication 3, **caractérisée en ce que** la quantité de buprénorphine est la quantité maximale ou respectivement la quantité minimale d'une dose individuelle et/ou la quantité maximale ou minimale administrée par jour.

5. Utilisation suivant l'une des revendications 1 à 4, **caractérisée en ce que** le médicament montre une libération retardée, et se présente avantageusement sous forme d'une formulation retard.

6. Utilisation suivant la revendication 5, **caractérisée en ce que** le médicament se présente sous la forme d'une particule ou d'un implant à libération retardée, en particulier d'un implant ou d'une particule en matière plastique, la matière plastique étant avantageusement choisie entre un polylactide, un polyglycolide ou un copolymère polylactide/polyglycolyde.

7. Utilisation suivant la revendication 5, **caractérisée en ce que** le médicament produit est un système thérapeutique transdermique sous forme d'un emplâtre destiné à l'administration du buprénorphine sur la peau.

8. Utilisation suivant la revendication 7, **caractérisée en ce que** le système thérapeutique transdermique est constitué d'une couche arrière laissant passer la substance active, d'une couche réservoir adhésive et d'une couche de protection pouvant être décollée.

9. Utilisation suivant la revendication 8, **caractérisée en ce que** la couche réservoir contient 20 à 90 % en poids de matière polymère, 0,1 à 30 % en poids de plastifiant, 0,1 à 20 % en poids de buprénorphine également sous forme de ses racémates, de ses énantiomères, de ses diastéréoisomères, en particulier sous forme de mélanges de ses énantiomères ou diastéréoisomères ou sous forme d'un énantiomère ou diastéréoisomère individuel ; sous forme de sa base et/ou de ses sels d'acides acceptables du point de vue physiologique, avantageusement sous forme de la buprénorphine base, et 0,1 à 30 % en poids de solvant pour la buprénorphine, le solvant pour la buprénorphine restant dans le système dans la couche réservoir étant avantageusement un composé portant au moins un groupe acide.

10. Utilisation suivant l'une des revendications 1 à 9, **caractérisée en ce que** le médicament montre une vitesse de libération de la buprénorphine entre 1 µg/h et 40 µg/h, avantageusement entre 2 µg/h et 35 µg/h, en particulier entre 5 µg/h et 20 µg/h, notamment entre 5 µg/h et 10 µg/h.

11. Utilisation suivant l'une des revendications 7 à 9, **caractérisée en ce que** le système thérapeutique transdermique présente une vitesse de libération de la buprénorphine de premier ordre sur un intervalle de dosage de 72 heures, de sorte qu'une concentration maximale dans le plasma entre 20 pg/ml et 1052 pg/ml est atteinte, et **en ce que** lors du traitement, le système thérapeutique transdermique reste sur la peau du patient pendant un autre intervalle de dosage d'au moins 2 jours, pendant lequel le système thérapeutique transdermique présente une cinétique de libération de la buprénorphine d'ordre nul, si bien que les patients éprouvent une analgésie pendant l'intervalle de dosage additionnel d'au moins 2 jours.

12. Utilisation suivant la revendication 11, **caractérisée en ce que** pendant l'intervalle additionnel de dosage d'au moins 2 jours, une vitesse moyenne de libération comprise entre 0,3 µg/h et 21 µg/h, avantageusement entre 0,3 µg/h et 9 µg/h ou entre 13 µg/h et 21 µg/h, en particulier entre 0,3 µg/h et 0,6 µg/h, entre 0,7 µg/h et 1 µg/h, entre 2 µg/h et 4 µg/h, entre 4 µg/h et 7 µg/h ou 5 µg/h et 9 µg/h est entretenue.

13. Utilisation suivant l'une des revendications 11 ou 12, **caractérisée en ce que** le système thérapeutique transdermique montre une vitesse de libération de la buprénorphine de premier ordre sur un intervalle de dosage de 72 heures, si bien qu'environ 72 heures après l'application de ce système thérapeutique transdermique, une concentration moyenne dans le plasma entre 20 pg/ml et 1053 pg/ml, avantageusement entre 85 pg/ml et 263 pg/ml, en particulier entre 20 pg/ml et 66 pg/ml, entre 42 pg/ml et 132 pg/ml, entre 169 pg/ml et 526 pg/ml, entre 254 pg/ml et 789 pg/ml ou entre 339 pg/ml et 1052 pg/ml est atteinte.

14. Utilisation suivant l'une des revendications 11 à 13, **caractérisée en ce que** le système thérapeutique transdermique est laissé sur la peau du patient pendant au moins 5 jours.

15. Utilisation suivant l'une des revendications 1 à 14, **caractérisée en ce que** le médicament contient un antagoniste de la morphine, en particulier la naloxone, la naltrexone et/ou le lévallorphane, à côté de la buprénorphine.

16. Utilisation suivant l'une des revendications 1 à 15, **caractérisée en ce que** le médicament contient de la buprénorphine sous forme de la base libre, du chlorhydrate, du stéarate, du citrate ou du lactate, en particulier sous forme de la base libre ou du chlorhydrate.
